# EUROPEAN PATENT APPLICATION

(11) **EP 4 640 169 A1**
(43) Date of publication of application: **29.10.2025**
(21) Application number: 23907139.2
(22) Date of filing: 21.12.2023
(51) Int. Cl.: A61B 17/29, B25J 18/06

(54) **BENDING STRUCTURE AND SURGICAL TOOL USING SAME**

(30) Priority: 23.12.2022 JP 2022206889
(71) Applicant: NHK Spring Co., Ltd., Yokohama-shi, Kanagawa 236-0004 (JP)
(72) Inventor: HAYAKAWA, Yuki, Yokohama-shi, Kanagawa 236-0004 (JP); HIRATA, Takafumi, Yokohama-shi, Kanagawa 236-0004 (JP); ISHIYAMA, Yuta, Yokohama-shi, Kanagawa 236-0004 (JP)
(74) Representative: Becker, Eberhard
(86) International application number: PCT/JP2023/045937
(87) International publication number: WO 2024/135779

(57) **Abstract**

Provided is a bending structure capable of achieving a reduced diameter. The bending structure comprises: a bending part 11 which performs bending and stretching; a movable part 13 which is provided at a movable side end that operates according to the bending and stretching of the bending part 11; an operation wire 17 which is inserted through the bending part 11 and the movable part 13, has an end 17a on the movable part 13, and is for causing the bending and stretching; and a case part 27 which is mounted on the movable part 13 and holds the end 17a of the operation wire 17 on the movable part 13.

## Description

### Technical Field

The present invention relates to a bending structure supplied to robots, manipulators etc. and a surgical tool using the same.

### Description of Related Art

As a conventional bending structure, for example, there exists one described in Patent Literature 1.

In the bending structure, a movable part is provided at the tip of an outer tube as a bending part that performs bending and stretching. The outer tube is made to perform bending and stretching by a drive wire as an operation wire. The operation wire is inserted through the inside of the outer tube, and an end part is supported by the movable part. The support of the end part is performed by having the entire end part stored in a connection hole provided in the movable part.

In such a structure, even if the drive wire breaks, the end part of the drive wire can be held in the connection hole of the movable part.

However, when reducing the outer diameter of the bending structure, there may be a need to open the connection hole to the outer circumference of the movable part, and the end part may fall off at wire breakage of the drive wire. Therefore, in the conventional bending structure, there was a limit to reduction in a diameter.

### Related Art

### Patent Literature

Patent Literature 1: Japanese Patent Application Laid-Open No. 2022-073298

### SUMMARY

### Technical Problem

The problem to be solved is that there was a limit to reduction in a diameter of the bending structure.

### Solution to the Problem

The present invention provides a bending structure which includes: a bending part that performs bending and stretching; a movable part that is provided at a movable side end part that operates according to the bending and stretching of the bending part; an operation wire for performing the bending and stretching that is inserted through the bending part and the movable part and has an end part positioned on the movable part; and a holding member that is mounted on the movable part and holds the end part of the operation wire on the movable part.

Moreover, the present invention provides a surgical tool using the bending structure, the surgical tool includes an end effector mounted on the bending structure, and the end effector includes a case part that is mounted on the movable part and functions as the holding member. Effects

According to the present invention, reducing a diameter of the bending structure may be achieved.

### BRIEF DESCRIPTION OF THE DRAWINGS

FIG. 1 is a front view showing forceps as a surgical tool using a bending structure according to Example 1 of the present invention.
FIG. 2 is a cross-sectional view showing the forceps of FIG. 1.
FIG. 3(A) is an enlarged cross-sectional view showing the vicinity of the movable part of the forceps of FIG. 2, and FIG. 3(B) is a cross-sectional view shifted by 90° in the circumferential direction with respect to FIG. 3(A).
FIG. 4 is a perspective view of the movable part of FIG. 3 as seen from the surface side.
FIG. 5 is a perspective view of the movable part of FIG. 3 as seen from the bottom side.
FIG. 6 is a plan view of the movable part of FIG. 3.
FIG. 7 is a bottom view of the movable part of FIG. 3.
FIG. 8 is a perspective view showing the vicinity of the movable part of the bending structure of FIG. 2.
FIG. 9 is a plan view showing the vicinity of the movable part of FIG. 8.
FIG. 10 is a perspective view showing the case part of the end effector of the forceps of FIG. 1.
FIG. 11 is a side view of the case part of FIG. 10.
FIG. 12 is a cross-sectional view taken along the line XII-XII of FIG. 11.
FIG. 13 is a perspective view showing the jaw holder of the end effector of the forceps of FIG. 2.

### DESCRIPTION OF EMBODIMENTS

The purpose of enabling the reduction in a diameter of a bending structure is realized by holding the end part of an operation wire on a movable part with a holding member mounted on the movable part.

As shown in the figures, a bending structure 5 includes a bending part 11, a movable part 13, an operation wire 17, and a holding member 27.

The bending part 11 is a part that performs bending and stretching. The movable part 13 is a member provided at a movable side end part that operates in response to the bending and stretching of the bending part 11. The operation wire 17 is a member for performing bending and stretching, which is inserted through the bending part 11 and the movable part 13, and has an end part 17a positioned on the movable part 13. The holding member 27 is a member mounted on the movable part 13 and holds the end part 17a of the operation wire 17 on the movable part 13.

The holding member 27 may be anything that holds the end part 17a of the operation wire 17, and may be a cover or clip etc. that is attached later to the movable part 13 or may be provided integrally therewith. In the case of being provided integrally, for example, the holding member may be joined to the movable part 13 by a hinge etc. In any case, the holding member 27 may have concave parts 35a and 35b that perform holding between the holding member 27 and the movable part 13.

The movable part 13 includes multiple circumferential parts 21a and 21b having different dimensions in an axial direction, and the operation wire 17 may be provided in plurality in a circumferential direction, with each of the end parts 17a positioned on the circumferential parts 21a and 21b.

The bending structure 5 may include a core material 15. The core material 15 is positioned within the bending part 11, with one end part positioned within the movable part 13. In this case, the movable part 13 may hold the one end part of the core material 15 with a circumferential part 21a having a relatively large dimension in the axial direction.

Such a bending structure 5 is applicable to various robots and manipulators, but can be used, for example, in a surgical tool.

The surgical tool includes an end effector 7 that is mounted on the bending structure 5. The end effector 7 is mounted on the movable part 13 of the bending structure 5, and may include a case part 27 that functions as a holding member.

The end effector 7 can adopt various types, but may be one including a pair of jaws 29 and a jaw holder 31. The pair of jaws 29 are ones that open and close between each other. The jaw holder 31 rotatably supports at least one of the jaws 29, and is supported inside the case part 27. In the case of single-opening, one of the pair of jaws 29 is rotatably supported by the case part 27, and in the case of double-opening, both are rotatably supported by the case part 27.

The jaw holder 31 is positioned on the end part 17a of the operation wire 17 located in the circumferential part 21b having a relatively small dimension in the axial direction, and may be adjacent in the radial direction to the end part 17a of the operation wire 17 located in the circumferential part 21a having a relatively large dimension in the axial direction.

### Example 1

### [Forceps]

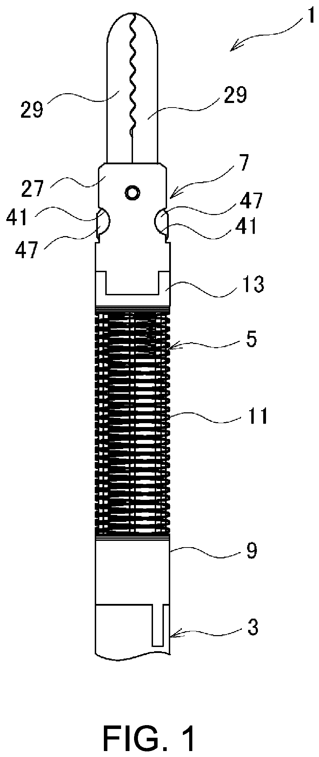

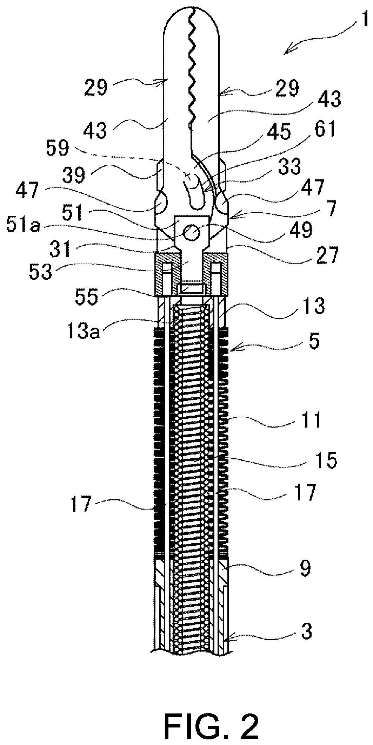

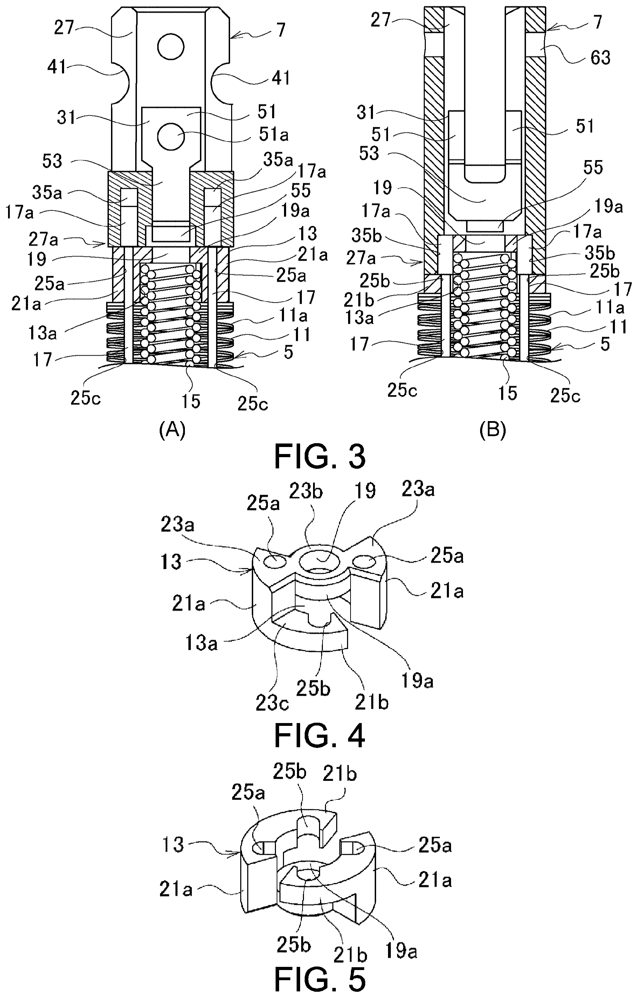

FIG. 1 is a front view showing forceps as a surgical tool using a bending structure according to Example 1 of the present invention. FIG. 2 is a cross-sectional view showing the forceps of FIG. 1. FIG. 3(A) is an enlarged cross-sectional view showing the vicinity of the movable part of the forceps of FIG. 2, and FIG. 3(B) is a cross-sectional view shifted by 90° in the circumferential direction with respect to FIG. 3(A).

The forceps 1 of the example, as shown in FIGS. 1 and 2, is a surgical tool used in surgical assistance robots and surgical assistance manipulator etc., and includes a shaft 3, the bending structure 5, and the end effector 7.

Moreover, in the example, forceps 1 are shown as an example of a surgical tool, but the surgical container tool to which the bending structure 5 can be applied is not limited to the forceps 1 as long as the surgical tool performs bending and stretching. In addition, the bending structure 5 can be applied to various devices such as robots and manipulator etc. that perform bending and stretching, other than surgical tools.

The shaft 3 is formed in a cylindrical shape, and is, for example, coupled to a surgical assistance robot or surgical assistance manipulator etc. in a manner allowing rotation around the spindle. At the tip of the shaft 3, the bending structure 5 is provided. It should be noted that the shape of the shaft 3 is not particularly limited.

The bending structure 5 is a part that has joint function and enables bending and stretching in the forceps 1. The bending structure 5 of the example includes a base part 9, the bending part 11, the movable part 13, the core material 15, and the operation wire 17.

The base part 9 is a configuration that receives one end (fixed side end part) of the bending part 11. The base part 9 is a columnar body, particularly a stepped cylindrical body, formed of resin or metal etc. The base part 9 is mounted by fitting to the tip of the shaft 3. One end of the bending part 11 is mounted on the base part 9 by welding etc. However, one end of the bending part 11 may also just contact the base part 9.

Moreover, the base part 9 receives one end of the bending part 11 in the axial direction, and as long as the base part 9 can be coupled to the end part of the shaft 3, the material, shape, and structure can be freely set according to the device to which the bending structure 5 is applied. The axial direction refers to the direction along the axial center of the forceps 1.

The bending part 11 is, as shown in FIG. 2 to FIG. 3(B), formed by laminating multiple wave washers 11a in the axial direction and maintaining the laminated state by welding etc. The bending part 11 operates such that the other end (movable side end part) in the axial direction moves relative to one end in the axial direction that is received by the base part 9 through bending and stretching.

Moreover, the bending part 11 is not particularly limited as long as the bending part 11 is capable of bending and stretching, and may also be made of a coil spring or bellows etc. At the tip of the bending part 11, the movable part 13 is provided.

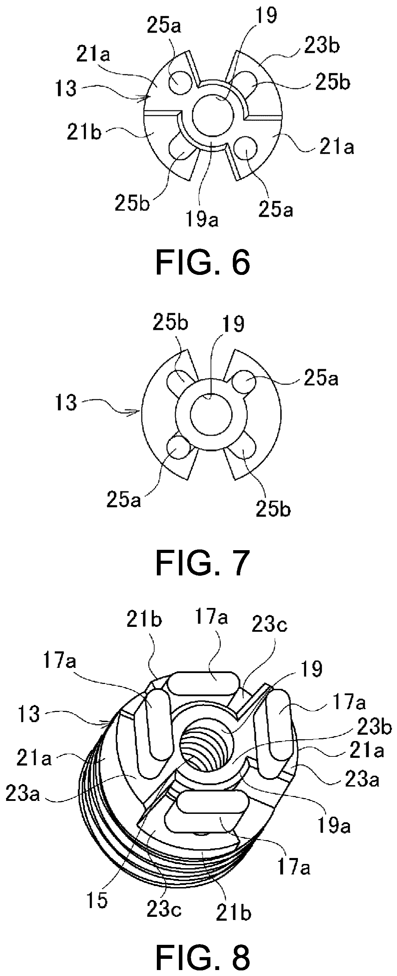

FIG. 4 is a perspective view of the movable part as seen from the surface side, FIG. 5 is a perspective view of the movable part as seen from the bottom side, FIG. 6 is a plan view of the movable part, and FIG. 7 is a bottom view of the movable part.

The movable part 13 is, as shown in FIGS. 3(A) and (B), configured to receive the other end (movable side end part) in the axial direction of the bending part 11. The movable part 13 is formed of resin or metal etc. as a columnar body, particularly a cylindrical body. In the example, the movable part 13 is placed on the other end of the bending part 11, and is mounted on the other end of the bending part 11 by welding etc. However, the movable part 13 may also merely be in contact with the other end of the bending part 11.

Moreover, the movable part 13 can be established freely in terms of material, shape, and structure according to the device to which the bending structure 5 is applied, as long as the movable part 13 can receive the other end of the bending part 11 in the axial direction.

The movable part 13 of the example, as shown in FIGS. 3(A) to 7, has a through hole 19 in the axial direction provided in the center part. Around the through hole 19, there are provided multiple circumferential parts 21a and 21b having different dimensions in the axial direction. In the example, the circumferential part 21a has a relatively large dimension in the axial direction, and the circumferential part 21b has a relatively small dimension in the axial direction.

In the example, the circumferential parts 21a are integrally provided in two at an edge part 19a of the through hole 19. The circumferential parts 21a are disposed at positions that differ by 180° in the circumferential direction. It should be noted that the number and disposition spacing of the circumferential parts 21a are optional, and one or three or more circumferential parts 21a may be provided. The edge part 19a of the through hole 19 is an annular portion that defines the through hole 19.

Each of the circumferential parts 21a exists as a columnar body with a fan-shaped ring in planar view. However, the planar shape of the circumferential part 21a is optional, and may be other shapes such as a rectangular shape etc. It should be noted that planar view refers to the state when viewed from the axial direction.

A surface 23a of the circumferential part 21a is configured to be flush with a surface 23b of the edge part 19a of the through hole 19. However, the surface 23a of the circumferential part 21a and the surface 23b of the edge part 19a do not need to be flush. The surface 23b of the edge part 19a and the surface 23a of the circumferential part 21a refer to the surfaces on the opposite side of the bending part 11 in the axial direction.

Such a circumferential part 21a protrudes from the edge part 19a of the through hole 19 toward the bending part 11 side in the axial direction. At the end part of the circumferential part 21a on the bending part 11 side, the circumferential part 21b is provided.

The circumferential parts 21b are integrally provided in two on each of the circumferential parts 21a. Accordingly, the circumferential parts 21b are disposed at positions that differ by 180° in the circumferential direction. It should be noted that the number and disposition spacing of the circumferential parts 21b can also be optionally set according to the circumferential part 21a.

Each of the circumferential parts 21b has a plate shape that is a fan-shaped ring when viewed in plan view. The planar shape of the circumferential part 21b is optional as far as, and may be other shapes such as a rectangular shape etc. A surface 23c of the circumferential part 21b is positioned on the bending part 11 side in the axial direction with respect to the surface of the circumferential part 21a.

There is a gap between the circumferential part 21b and the edge part 19a of the through hole 19 in the axial direction. In this way, an internal part 13a of the movable part 13 is open to the outside. The internal part 13a of the movable part 13 becomes a space part into which one end part of the core material 15 is inserted. In the circumferential direction, the circumferential part 21b protrudes from the circumferential part 21a, and is disposed with spacing from the adjacent circumferential part 21b.

Such a movable part 13 is provided with insertion holes 25a and 25b for inserting the operation wire 17 through the circumferential parts 21a and 21b, respectively. The insertion holes 25a and 25b penetrate through the circumferential parts 21a and 21b in the axial direction, respectively.

The insertion hole 25a is a hole that is closed in a plan view between the circumferential part 21a and the edge part 19a of the through hole 19. Also, the insertion hole 25a is a concave-shaped hole in a plan view in which the radial inner side is open in a portion in which the circumferential part 21a protrudes in the axial direction from the edge part 19a of the through hole 19. The insertion hole 25b is, as a whole, a concave-shaped hole in a plan view in which the radial inner side is open. Here, the radial direction refers to the direction along the diameter of the forceps 1.

The core material 15, as shown in FIGS. 2 and 3, is a member capable of bending and stretching together with the bending part 11. The core material 15 in the example is a multiple coil spring, specifically a double coil spring. However, the core material 15 can also be a close-wound coil spring or a flexible columnar body etc.

The multiple coil spring is one in which the coil part of one of at least two coil springs is fitted and disposed between the coil parts of the other, and is capable of suppressing compression of the bending part 11. The multiple coil spring may also be one that uses three or more coil springs.

Such a core material 15 is inserted through the base part 9 and the bending part 11, and one end is positioned in the internal part 13a of the movable part 13. In this state, one end of the core material 15 is pushed against the edge part 19a of the through hole 19 in the axial direction, and is held in the radial direction by the circumferential part 21a.

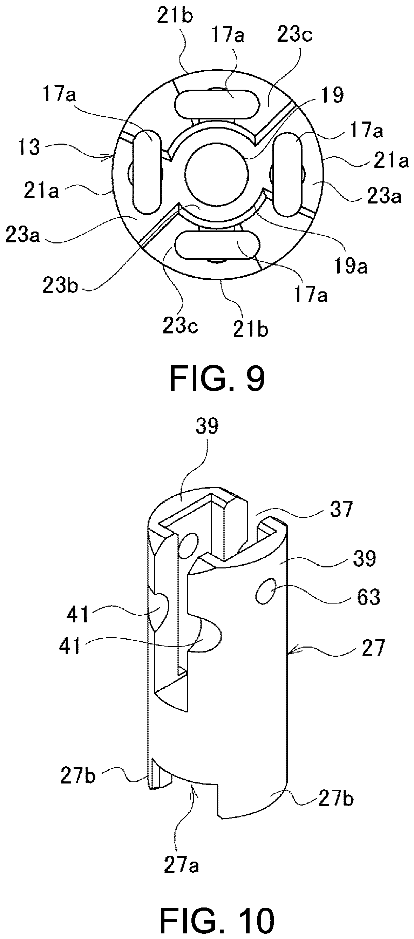

FIG. 8 is a perspective view showing the vicinity of the movable part of the bending structure, and FIG. 9 is a plan view showing the vicinity of the movable part of FIG. 8.

The operation wire 17 of FIGS. 2 to 3(B) is for making the bending part 11 perform bending and stretching by being pulled in the axial direction. The operation wires 17 of the example are provided at four places in the circumferential direction of the bending structure 5 at 90 degree intervals. When one or more of the operation wires 17 is pulled toward the base part 9 side against the movable part 13, the bending part 11 can be bent to make the movable part 13 operate.

Furthermore, operation in the axial direction means performing relative pulling and retracting of the operation wire 17 in the axial direction. The number and disposition spacing of the operation wires 17 can be optionally set.

Each of the operation wires 17 can be constituted of stranded wire, single wire, piano wire, multi-joint rod, chain, cord, thread, rope etc. The operation wire 17 is inserted through the bending part 11 and the movable part 13, and has the end part 17a positioned on the movable part 13.

That is, the operation wire 17 is inserted through the insertion hole 25c provided in the bending part 11, and is inserted through the insertion hole 25a or 25b in the movable part 13. The end part 17a of the operation wire 17 is one to which an end processing member such as a sleeve is attached by crimping etc.

The end part 17a, as shown in FIGS. 3(A) and (B) as well as FIGS. 8 and 9, has a shape that protrudes in a plan view compared to other parts of the operation wire 17. As a result, the end part 17a protrudes in a plan view beyond the through holes 25a and 25b of the movable part 13, and is placed on the surfaces 23a and 23c of the circumferential parts 21a and 21b of the movable part 13. Therefore, the end part 17a is positioned on the movable part 13.

Moreover, the end part 17a, in addition to being placed on the surfaces 23a and 23c of the movable part 13, also includes a state where a portion enters into a concave part provided in the axial direction on the circumferential parts 21a and 21b. The concave part has the through holes 25a and 25b opening at the bottom part thereof.

Such an end part 17a of the operation wire 17 is held by the case part 27 of the end effector 7, which is a holding member.

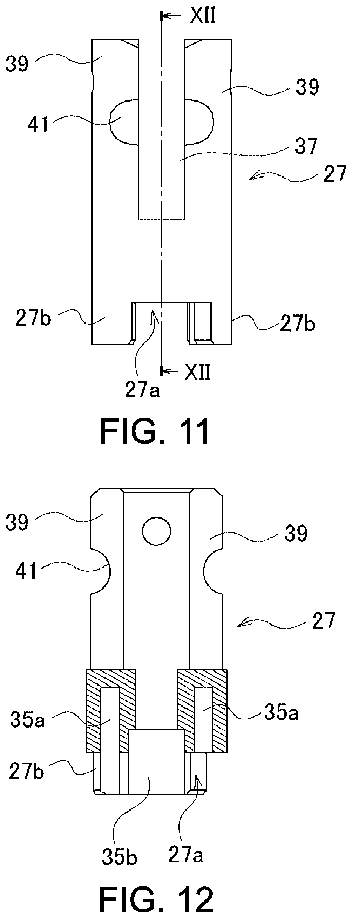

FIG. 10 is a perspective view showing the case part, FIG. 11 is a side view of the case part of FIG. 10, and FIG. 12 is a cross-sectional view taken along the line X-X of FIG. 11.

The end effector 7 of the example, as shown in FIGS. 1 to 3(B), includes the case part 27, the pair of jaws 29, the jaw holder 31, and a cam part 33. It should be noted that the end effector 7 has the pair of jaws 29 that open and close by a cam type, but may also be of a link type.

The case part 27 is mounted on the movable part 13, and functions as a holding member. The holding member refers to that which is mounted on the movable part 13 and holds the end part 17a of the operation wire 17 on the movable part 13. The holding here refers to holding in the radial direction and axial direction, and means to the extent that the end part 17a does not fall off when the operation wire 17 breaks. Furthermore, the holding member may also be a cover or clip etc. separate from the case part 27.

The case part 27, as shown in FIGS. 3(A) and (B) and FIGS. 10 to 12, is formed as a whole in a cylindrical shape, but is not particularly limited thereto as long as the case part 27 is mounted on the movable part 13. In the example, the case part 27 has a one end part 27a in the axial direction with a shape corresponding to the movable part 13. The one end part 27a fits into the movable part 13. As a result, the one end part 27a of the case part 27 is pushed against the surfaces 23a and 23c of the movable part 13. Furthermore, the case part 27 may have a shape that covers the entire movable part 13.

The one end part 27a of the case part 27 has the concave parts 35a and 35b that perform holding between the one end part 27a and the movable part 13.

The concave part 35a is, in a plan view, a concave shape in the axial direction that is closed. The concave part 35a holds the end part 17a of the operation wire 17 on the circumferential part 21a by placing the end part 17a inside.

The concave part 35b is provided on the inner side in the radial direction of a protrusion part 27b in the axial direction of the one end part 27a of the case part 27. The concave part 35b is formed as a concave shape in the axial direction and radial direction. The concave part 35b holds the end part 17a on the circumferential part 21b by pressing the end part 17a from the outer side in the radial direction and axial direction. Although the concave part 35b does not perform holding of the end part 17a from the inner side in the radial direction, the holding is performed by the edge part 19a of the through hole 19, as shown in FIGS. 8 and 9.

Therefore, even if the operation wire 17 breaks, the dropping of the end part 17a can be suppressed. Also, since it is not needed to hold the end part 17a of the operation wire 17 on the movable part 13 side, reducing the diameter of the bending structure 5 may be achieved.

Such a case part 27, as shown in FIGS. 1 and 2, rotatably supports the pair of jaws 29 to make the pair of jaws 29 openable and closable. The case part 27 of the example, as shown in FIGS. 10 to 12, has a slit 37 provided at the tip side in the axial direction, and support wall parts 39 that support the jaws 29 are defined on one side and the other side in the radial direction across the slit 37.

The case part 27 has support concave parts 41 that open respectively on both sides in the radial direction along the slit 37.

The pair of jaws 29 in FIGS. 1 and 2 are configured to open and close between each other, and in the example, the pair of jaws 29 are constructed as grippers that perform grasping through closing operation. The jaws 29 are formed in the same shape and are disposed with front and back sides thereof reversed. However, the jaws 29 do not need to be of the same shape. Each of the jaws 29 includes a main body part 43, an arm 45, and a rotation axis part 47.

The main body part 43 is formed in a rod shape, and is a portion that performs opening and closing operation. At the base end in the axial direction of the main body part 43, the rotation axis part 47 is provided through the arm 45.

The rotation axis part 47 is rotatably engaged by sliding in the support concave part 41 of the case part 27. The shapes of the rotation axis part 47 and the support concave part 41 can be optionally set as far as the rotation axis part 47 can be rotatably supported around the spindle by sliding in the support concave part 41.

The arm 45 is formed in a plate shape. The arm 45 is inserted into the slit 37 of the case part 27 shown in FIGS. 10 and 11. The arm 45 is coupled to the jaw holder 31, and enables opening and closing operation of the jaws 29 to be performed by the jaw holder 31.

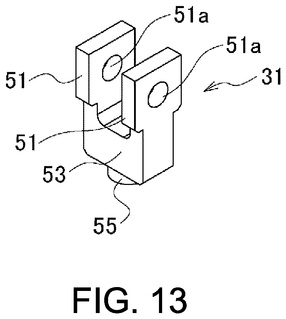

FIG. 13 is a perspective view showing the jaw holder.

The jaw holder 31 is a member that is rotatably coupled to both of the pair of jaws 29 in FIGS. 1 and 2, and is capable of reciprocating movement relative to the case part 27. The jaw holder 31, as shown in FIG. 13, has opposing holder plate parts 51. Between the holder plate parts 51, the arms 45 of the pair of jaws 29 are disposed.

Each of the holder plate parts 51 of the jaw holder 31 is provided with an opening part 51a. Corresponding to the opening part 51a, a hole (not shown) is provided in the arm 45 of the jaw 29. A spindle 49 is inserted across the hole of the arm 45 and the opening part 51a. Therefore, the jaws 29 are rotatably engaged with the jaw holder 31.

A connection part 55 that protrudes in the axial direction is provided on the holder base 53 that combines the holder plate parts 51. The connection part 55 is formed in a tubular shape, and an operation member (not shown) such as a push-pull cable etc. is connected to the connection part 55. By the operation member, the jaw holder 31 can perform reciprocating movement in the axial direction.

By the reciprocating movement, the jaw holder 31 exerts a force around the rotation center to the jaws 29 through the spindle 49, which is the connecting portion.

Such a jaw holder 31, as shown in FIGS. 3(A) and (B), is positioned on the end part 17a of the operation wire 17 located in the circumferential part 21b having a relatively small dimension in the axial direction within the case part 27. Also, the jaw holder 31 is adjacent in the radial direction to the end part 17a of the operation wire 17 located in the circumferential part 21a having a relatively large dimension in the axial direction within the case part 27.

Therefore, in the example, the jaw holder 31 can be disposed in a biased manner toward the movable part 13 in the axial direction, and the dimension in the axial direction of the forceps 1 may be reduced.

The cam part 33, as shown in FIG. 2, is provided between the case part 27 and the jaws 29, and rotates the jaws 29 to open and close the jaws 29 in response to the reciprocating movement of the jaw holder 31.

The cam part 33 can adopt various forms, but in the example, the cam part 33 consists of a cam pin 59 mounted on the case part 27 and a cam groove 61 provided in the jaw 29.

The cam pin 59 is mounted in the through hole 63 of the case part 27 shown in FIG. 10, and a tip thereof protrudes into the slit 37 of the case part 27. Within the slit 37, as shown in FIG. 2, the tip of the cam pin 59 engages with the cam groove 61. The cam pin 59 is of a cylindrical shape, but is not particularly limited thereto.

The cam groove 61 is provided in the arm 45 of each of the jaws 29. The cam groove 61 is formed in an arc shape around the rotation center. It should be noted that the cam groove 61 consists of a concave part, but may also be a through hole. By the cam pin 59 relatively moving along the cam groove 61, the opening and closing operation is performed on the jaws 29.

### [Assembly]

When assembling the forceps 1 of the example, as shown in FIGS. 8 and 9, the operation wire 17 is assembled to the bending structure 5. The operation wire 17 is inserted through the movable part 13, and the end part 17a is positioned on the movable part 13.

The end effector 7 is mounted on the bending structure 5, as shown in FIGS. 3(A) and (B).

When mounting the end effector 7, the movable part 13 of the bending structure 5 is fitted into the one end part 27a of the case part 27. As a result, the one end part 27a of the case part 27 is pushed against the movable part 13. At this time, the concave parts 35a and 35b of the one end part 27a of the case part 27 engage with the end part 17a on the movable part 13 and hold the end part 17a between the concave parts 35a and 35b and the movable part 13.

In this way, in the example, the end part 17a of the operation wire 17 can be held by the case part 27 of the end effector 7, which is the holding member. Therefore, it is not needed to hold the end part 17a of the operation wire 17 on the movable part 13 side, reducing the diameter of the bending structure 5 may be achieved.

The holding of the end part 17a of the operation wire 17 is performed by the concave parts 35a and 35b provided in the case part 27, so the holding can be easily performed when mounting the case part 27.

Also, in the example, by utilizing the case part 27 as a holding member, the number of parts can be reduced and the configuration can be simplified.

In a state where the end effector 7 is mounted on the bending structure 5, the end part of the core material 15 of the bending structure 5 is positioned in the internal part 13a of the movable part 13. In the example, the end part of the core material 15 can be held by the circumferential part 21a which has a relatively large dimension in the axial direction.

Moreover, in the example the jaw holder 31 of the end effector 7 is positioned on the end part 17a of the operation wire 17 located at the circumferential part 21b having a relatively small dimension in the axial direction, and is adjacent in the radial direction to the end part 17a of the operation wire 17 located at the circumferential part 21a. Therefore, the dimension of the forceps 1 in the axial direction can be reduced.

### Reference Signs List

1: forceps (surgical tool)
3: shaft
5: bending structure
7: end effector
9: base part
11: bending part
13: movable part
15: core material
17: operation wire
17a: end part
21a, 21b: circumferential part
27: case part
29: jaw
31: jaw holder
35a, 35b: concave part

## Claims

1. A bending structure, comprising:
a bending part, performing bending and stretching;
a movable part, provided at a movable side end part that operates according to the bending and stretching of the bending part;
an operation wire for performing the bending and stretching, being inserted through the bending part and the movable part, and comprising an end part positioned on the movable part; and
a holding member, mounted on the movable part, and holding the end part of the operation wire on the movable part.

2. The bending structure according to claim 1, wherein
the holding member comprises a concave part that performs holding between the holding member and the movable part.

3. The bending structure according to claim 1 or 2, wherein
the movable part comprises a plurality of circumferential parts having different dimensions in an axial direction, and
the operation wire is provided in plurality in a circumferential direction, each having the end part positioned on the circumferential part.

4. The bending structure according to claim 3, comprising:
a core material, positioned inside the bending part and having an one end part positioned inside the movable part, and
the movable part holding the one end part of the core material with the circumferential part having a relatively large dimension in the axial direction.

5. A surgical tool using the bending structure according to claim 1 or 2, comprising:
an end effector, mounted on the bending structure, and
the end effector comprising a case part that is mounted on the movable part and functions as the holding member.

6. The surgical tool according to claim 5, wherein
the end effector comprises
a pair of jaws that are opened and closed between each other, and
a jaw holder that rotatably supports at least one of the pair of jaws and is supported inside the case part.

7. The surgical tool according to claim 6, wherein
the movable part comprises a plurality of circumferential parts having different dimensions in an axial direction, and
the operation wire is provided in plurality in a circumferential direction, each having the end part positioned on the circumferential part, and
the jaw holder is positioned on the end part of the operation wire positioned on the circumferential part having a relatively small dimension in the axial direction, and is adjacent in a radial direction to the end part of the operation wire positioned on the circumferential part having a relatively large dimension in the axial direction.
